# FASCICULE DE BREVET EUROPEEN

(11) **EP 4 103 243 B1**
(45) Date de publication et mention de la délivrance du brevet: **30.10.2024**
(21) Numéro de dépôt: 21705128.3
(22) Date de dépôt: 09.02.2021
(51) Int. Cl.: A61L 9/013, B01D 11/02, C11D 3/00, C11D 3/20, C11D 3/34, D06M 13/00, A61L 9/04

(54) **COMPOSITION DESODORISANTE RENFERMANT DU 1,8-PARA-MENTHENETHIOL, DE L'ACETATE DE 3-MERCAPTOHEXYLE ET DE L'ACIDE UNDECYLENIQUE OU SES DERIVES**
DESODORIERENDE ZUSAMMENSETZUNG, DIE 1,8-PARA-MENTHENETHIOL, 3-MERCAPTOHEXYLACETAT UND UNDECYLENSÄURE ODER DIE DERIVATE DAVON ENTHÄLT
DEODORANT COMPOSITION CONTAINING 1,8-PARA-MENTHENETHIOL, 3-MERCAPTOHEXYL ACETATE AND UNDECYLENIC ACID OR THE DERIVATIVES THEREOF

(30) Priorité: 10.02.2020 FR 2001264
(43) Date de publication de la demande: 21.12.2022
(73) Titulaire: Jafer Enterprises R&D SL, 08403 Granollers (ES)
(72) Inventeur: GOUTEYRON, Antoine, 06110 LE CANNET (FR); PEREZ, Marion, 06110 LE CANNET (FR); CHARIER, Chloé, 06600 ANTIBES (FR); DELMAS, Thomas, 06600 ANTIBES (FR)
(74) Mandataire: Renard, Emmanuelle
(86) Numéro de dépôt international: PCT/EP2021/053091
(87) Numéro de publication internationale: WO 2021/160613

(56) Documents cités:
- WO-A1-2018/167206
- US-A1- 2011 300 095

## Description

### OBJET DE L'INVENTION

La présente invention concerne une composition désodorisante renfermant : (a) du 1,8-para-menthènethiol, (b) de l'acétate de 3-mercaptohexyle et (c) de l'acide undécylénique ou l'un de ses dérivés. Elle a également pour objet l'utilisation de cette composition pour éliminer les mauvaises odeurs, ainsi qu'un produit désodorisant sous forme d'aérosol, de bougie ou de diffuseur de parfum à mèche, à bâtonnets ou électrique, ou d'article constitué d'un matériau polymérique solide, notamment d'un élastomère de silicone, contenant la composition précitée.

### ARRIERE-PLAN DE L'INVENTION

De nombreux produits capables de réduire ou masquer les mauvaises odeurs sont disponibles dans le commerce et ont été décrits dans la littérature. Ces produits désodorisants agissent soit par voie physique, en piégeant les molécules responsables des mauvaises odeurs dans des filtres ayant une porosité et une surface spécifique adaptées, notamment des cyclodextrines, soit par voie physico-chimique, en réduisant la volatilité des composés malodorants, soit encore par voie sensorielle, en masquant les mauvaises odeurs à l'aide de parfums qui permettent de réduire leur perception. Parmi ces derniers, la Demanderesse a déjà proposé d'utiliser la combinaison du 1,8-para-menthènethiol ou thioterpinéol avec l'acétate de 3-mercaptohexyle, qui est notamment présente dans certains extraits végétaux et en particulier dans une composition volatile extraite de *Zanthoxylum armatum* (WO 2018/167206).

La Demanderesse a ainsi envisagé l'utilisation de ces extraits comme agents masqueurs d'odeurs dans différents produits désodorisants.

Bien que ces extraits se soient avérés très efficaces dans le masquage d'odeurs de diverses origines, il est apparu que ces odeurs pouvaient être éliminées plus rapidement en associant les constituants de ces extraits à l'acide undécylénique ou ses dérivés. La Demanderesse a en effet constaté que cette association permettait non seulement de masquer les mauvaises odeurs, mais également de limiter leur diffusion dans l'atmosphère et donc de les neutraliser.

L'utilisation de l'acide undécylénique ou de l'un de ses dérivés comme agent désodorisant a déjà été décrite dans plusieurs documents, notamment US2011/300095 ; US 5,976,460; US 5,275,783 ; US 5,338,511 ; et US 5,439,641. A la connaissance de la Demanderesse, il n'a toutefois encore jamais été suggéré de les associer à une combinaison de 1,8-para-menthènethiol et d'acétate de 3-mercaptohexyle, ou à un extrait végétal renfermant cette combinaison. Par ailleurs, il n'était pas prévisible que l'acide undécylénique ou ses dérivés permettent d'améliorer l'efficacité de la combinaison précitée, a fortiori de manière synergique, dans la mesure où il est bien connu que le mélange de molécules désodorisantes peut présenter un effet antagoniste de celui obtenu lorsque ces molécules sont utilisées individuellement.

### RESUME DE L'INVENTION

L'invention a ainsi pour objet une composition désodorisante renfermant : (a) du 1,8-para-menthènethiol, (b) de l'acétate de 3-mercaptohexyle et (c) de l'acide undécylénique ou l'un de ses dérivés.

Elle a également pour objet l'utilisation de cette composition pour éliminer les mauvaises odeurs.

Elle a encore pour objet un produit désodorisant sous forme d'aérosol, de bougie, de diffuseur de parfum à mèche, à bâtonnets ou électrique, ou d'article constitué d'un matériau polymérique solide, notamment d'un élastomère de silicone, contenant la composition précitée.

### DESCRIPTION DETAILLEE

L'invention porte sur une nouvelle composition adaptée à l'élimination des mauvaises odeurs, qui permet notamment de prévenir efficacement leur diffusion dans l'atmosphère. Cette composition comprend précisément trois constituants particuliers, à savoir (a) du 1,8-para-menthènethiol, (b) de l'acétate de 3-mercaptohexyle et (c) de l'acide undécylénique ou l'un de ses dérivés.

Les constituants (a) et (b) précités peuvent être introduits individuellement dans la composition ou être apportés par un ou plusieurs extraits végétaux qui les contiennent à l'état naturel. Selon une forme d'exécution, la composition selon l'invention renferme donc un extrait végétal ou un mélange d'extraits végétaux, tel qu'un extrait de Timur (*Zanthoxylum armatum*). Il s'agit d'un végétal qui croît en Asie et en particulier au Népal, dont les baies constituent le poivre de Timut. L'extrait utilisé selon l'invention peut être obtenu à partir de toute partie du Timur et notamment de ses feuilles, de son tronc, de ses fruits (baies) ou de leur péricarpe. Plus préférentiellement, on utilise un extrait de baies de Timur.

Dans un mode de réalisation préféré de cette forme d'exécution de l'invention, les baies de Timur peuvent être séchées et/ou broyées par tout moyen préalablement à leur extraction, notamment par cryobroyage. Il a en effet été observé que les baies broyées présentaient un meilleur équilibre entre leurs différentes notes olfactives, dû notamment à une modification du ratio monoterpènes / linalool. La granulométrie moyenne (D50) des baies broyées peut notamment être comprise entre 200 et 600µm, telle que mesurée par tamisage.

Dans le cas où elle contient un extrait de Timur, la composition selon l'invention renferme en outre avantageusement au moins un autre constituant naturellement présent dans un tel extrait et qui peut être choisi parmi : le linalool, le limonène, le myrcène, le cinnamate de méthyle, le β-phellandrène, le 4-mercapto-4-méthyl-pentan-2-one, le 3-mercaptohexanol et un mélange de ceux-ci, de préférence un mélange de tous les constituants précités. Selon une forme d'exécution de l'invention, cet extrait renferme de 5 à 99% de produits volatils et semi-volatils qui contiennent de 50 à 95% en poids d'une combinaison de linalool, limonène, myrcène et cinnamate de méthyle. Par « produit volatil », on entend un produit possédant une pression de vapeur inférieure à 13,34 Pa (0,1 mm Hg) à 20°C ou un point d'ébullition inférieur à 216°C. Par « produit semi-volatil », on entend un produit possédant un point d'ébullition compris entre 216 et 350°C.

Les composés (a) et (b) utilisés selon l'invention étant également présents séparément dans d'autres extraits végétaux que ceux issus du Timur, la composition selon l'invention peut en variante renfermer un mélange d'extraits végétaux, par exemple d'un mélange d'extrait de raisin noir, de fruit de la passion ou de houblon (contenant de l'acétate de 3-mercaptohexyle) et de pamplemousse, de citronnier épineux (*Pondras trifoliata*) ou d'orange (contenant du 1,8-para-menthènethiol).

L'extraction des parties de plante choisies pour obtenir le ou les extraits végétaux utilisés selon l'invention peut être effectuée par hydrodistillation ou préférentiellement à l'aide de CO₂ supercritique. Dans le cas du Timur, l'extraction au CO₂ supercritique permet d'obtenir un extrait présentant un parfum plus puissant, plus durable et moins riche en aldéhydes que l'extraction par hydrodistillation. Ces caractéristiques pourraient être liées à la plus grande richesse en acides gras et en esters de la fraction volatile ainsi obtenue. Les conditions de mise en oeuvre de ces procédés pourront être facilement déterminées par l'homme de l'art. Ainsi, l'extraction au CO₂ supercritique peut par exemple être effectuée à une température de 30 à 60°C, par exemple de 35 à 50°C, sous une pression de 50 à 150 bars, par exemple de 80 à 100 bars. L'extrait végétal obtenu peut éventuellement être séché par tout moyen, généralement jusqu'à une teneur en humidité de 0 à 10% en poids, avant d'être utilisé selon l'invention.

La présence de 1,8-para-menthènethiol et d'acétate de 3-mercaptohexyle dans l'extrait végétal peut être confirmée notamment par modification de ces thiols avec la 4,4'-dithiodipyridine ou le N-phénylmaléimide, respectivement, puis analyse par chromatographie en phase liquide à ultra haute performance (UHPLC) couplée à une analyse qualitative et quantitative par spectrométrie de masse (MS QqQ).

La composition selon l'invention renferme par exemple de 100 à 10.000 ppm, de préférence de 600 à 6.000 ppm, de 1,8-para-menthènethiol et généralement de 100 à 10.000 ppm, de préférence de 400 à 4.000 ppm d'acétate de 3-mercaptohexyle.

Un autre constituant essentiel de la composition selon l'invention est l'acide undécylénique ou l'un de ses dérivés. Les dérivés de l'acide undécylénique peuvent avantageusement être choisis parmi : un ester alkylique d'acide undécylénique, en particulier un ester d'alkyle linéaire ou ramifié, substitué ou non substitué, en C₁-C₁₂ tel qu'un ester de de méthyle, d'éthyle, de propyle, d'hexyle ou de décyle ; un ester d'acide polyoxyalkylénique, notamment un ester de polyoxyéthylène, polyoxypropylène ou poly(oxyéthylène)(oxypropylène) d'acide undécylénique, renfermant de préférence de 2 à 20 motifs polyoxyalkylène ; un sel d'acide undécylénique, en particulier un sel métallique, tel que les sels de sodium, potassium, magnésium, calcium, zinc ou cuivre ou en variante un sel d'ammonium d'acide undécylénique ; et leurs mélanges.

On préfère selon l'invention utiliser un ester alkylique d'acide undécylénique, en particulier l'undécylénate de méthyle.

La composition selon l'invention renferme par exemple de 0,001 à 10% en poids, de préférence de 0,01 à 5% en poids et plus préférentiellement de 0,1 à 1% en poids, d'acide undécylénique ou l'un de ses dérivés.

La composition selon l'invention peut être utilisée pour éliminer tous types de mauvaises odeurs, c'est-à-dire supprimer leur perception olfactive, en particulier en limitant leur diffusion dans l'air. Ces mauvaises odeurs peuvent notamment être choisies parmi des odeurs indolées, aminées (en particulier l'ammoniac et le skatole), thiolées (notamment le trisulfure de diméthyle ou le thiogéraniol ) ou acides (notamment l'acide isovalérique).

La composition selon l'invention peut par ailleurs servir à réduire ces odeurs dans tout type d'environnement dans lequel elle est appliquée, notamment sur le corps ou dans des locaux à usage domestique, commercial ou industriel. En variante, cette composition peut être combinée à des produits malodorants en eux-mêmes ou susceptibles de générer de mauvaises odeurs lors de leur utilisation.

Dans un premier mode de réalisation, la composition selon l'invention constitue un additif ajouté à un produit détergent tel qu'une lessive ou un nettoyant ménager (notamment un produit de nettoyage des toilettes ou de la vaisselle) ; à un produit d'hygiène corporelle tel qu'un déodorant, un anti-transpirant, un savon ou un shampooing ; ou à un produit de coloration capillaire, sans que cette liste ne soit limitative. Ces produits peuvent notamment se présenter sous forme de liquide, de gel, de mousse ou de crème ou encore sous forme solide. Selon une forme d'exécution particulière de ce mode de réalisation, le produit détergent peut être imprégné sur un produit non-tissé, notamment une lingette.

La composition selon l'invention peut être véhiculée dans des microsphères, des nanocapsules, des microcapsules, des liposomes ou tout autre vecteur permettant son incorporation dans les produits précités et éventuellement la libération prolongée de ses constituants. Un exemple préféré de microcapsules convenant à cet effet est constitué des microcapsules décrites dans le document FR 3 069 252. Elles renferment un coeur huileux comprenant la composition selon l'invention, ledit coeur étant revêtu d'une première membrane obtenue à partir d'au moins un polymère qui est de préférence choisi parmi les résines aminoplastes, de préférence les résines urée-formaldéhyde ou mélamine-formaldéhyde ou mélamine-urée-formaldéhyde, et les polyurées. La surface extérieure de la première membrane est recouverte d'une seconde membrane, qui est essentiellement constituée d'au moins un composé non siliconé insoluble dans l'eau, ayant un point de fusion compris entre 20 et 120°C. Ce composé est avantageusement choisi parmi les sels d'acide stéarique et d'acide ricinoléique, préférentiellement le stéarate de magnésium ou le ricinoléate de zinc, plus préférentiellement le stéarate de magnésium.

Dans un second mode de réalisation, la composition selon l'invention est utilisée dans la fabrication d'un produit désodorisant, qui peut notamment se présenter sous forme d'aérosol, de bougie, de diffuseur de parfum à mèche, à bâtonnets ou électrique, ou d'article constitué d'un matériau polymérique solide, notamment d'un élastomère de silicone. Ce produit est en particulier destiné à être utilisé comme désodorisant d'intérieur et/ou de textiles. La composition peut dans ce cas comprendre différents constituants permettant notamment au produit la contenant de se présenter sous une forme adaptée à son usage. Ces constituants peuvent par exemple comprendre des solvants tels que de l'eau et/ou des solvants organiques polaires ou apolaires, hydrocarbonés ou siliconés ; des agents tensioactifs ; des gélifiants de phase aqueuse ; des épaississants de phase huileuse ; des conservateurs ; des antioxydants ; des agents chélatants ; des polyols ; des absorbeurs d'odeurs tels que des zéolithes, des cyclodextrines, de la silice, des aluminosilicates ou du charbon actif ; des absorbeurs UV ; des agents antimicrobiens ; des parfums tels que des huiles essentielles ; des gaz propulseurs ; des pigments ; et des colorants, sans que cette liste ne soit limitative, pour autant que ces constituants ne nuisent pas aux propriétés désodorisantes de la composition selon l'invention.

Dans une forme d'exécution particulière de ce mode de réalisation, la composition renfermant les trois constituants décrits précédemment peut être imprégnée dans un article constitué d'un matériau polymérique solide, généralement d'un élastomère de silicone, comme décrit dans le document WO 2019/137894. Dans ce cas, la composition renferme en outre un solvant comprenant un monoester en C₁₀-C₁₈ d'alkyle en Ci-Cs, de préférence un monoester en C₁₂-C₁₆ d'alkyle en C₃-C₆, tel que le myristate d'isopropyle, avantageusement associé à un diester saturé en C₂-C₁₀ d'alkyle en C₃-C₁₂, de préférence un diester saturé en C₄-C₈ d'alkyle en C₆-C₁₀, plus préférentiellement l'adipate de dioctyle, afin d'améliorer le taux d'infusion de la composition désodorisante dans le matériau et son profil de diffusion. L'article obtenu peut par exemple se présenter sous forme d'anneau, de galet, de boule, de cube, de cylindre, de dôme ou de pyramide adaptés à être posés ou suspendus en vue de désodoriser l'atmosphère ou à être introduits dans le tambour d'une machine à laver.

Dans une autre forme d'exécution, la composition désodorisante selon l'invention peut être incorporée à un article absorbant, tel qu'une couche pour bébé, et en particulier dans le noyau absorbant et/ou l'une au moins des couches non tissées et/ou des films plastiques constituant les couches supérieure et inférieure du produit absorbant et/ou au sein d'élastiques.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Test de désodorisation sur cotons

On a préparé un réducteur de mauvaises odeurs (MOR) constitué d'un mélange de :
- 1,8-para-menthènethiol
- acétate de 3-mercaptohexyle
- undécylénate de méthyle.

Pour ce faire, on a d'abord dilué séparément le 1,8-para-menthènethiol (1,8-PMT) et l'acétate de 3-mercaptohexyle (3-HMA) à 0,00001% en poids dans du dipropylène glycol, puis les deux solutions ont été mélangées dans un rapport pondéral 1,8-PMT:3-HMA de 61:39 et l'ensemble a été dilué à 0,005% en poids dans l'éthanol. On a ainsi obtenu un mélange dit SSPS. De son côté, l'undécylénate de méthyle (UDM) a été dilué à 1% en poids dans l'éthanol. Le mélange SSPS et UDM a alors été mélangé dans un rapport pondéral SSPS:UDM de 20:80.

Le test consistait à évaluer la capacité désodorisante des réducteurs de mauvaises odeurs (MOR) suivants : SSPS, UDM, UDM + SSPS.

Pour ce faire, différentes mauvaises odeurs (ammoniac, thiogéraniol, diméthyl trisulfure) ont été injectées dans des flacons scellés en verre de 15 mL dans lesquels étaient placés un (ou deux pour l'ammoniac) carrés de coton. L'intensité olfactive de la mauvaise odeur (MO) a été évaluée par des panélistes entraînés après 2h30 d'équilibrage et la moyenne des résultats a été calculée. Le pourcentage de réduction de la mauvaise odeur est donné dans le Tableau 1 ci-dessous pour une certaine quantité de MOR vs. MO contre un flacon comportant uniquement la Mauvaise Odeur.

Dans ce tableau :
nSSPS / nMO = Concentration en moles de SSPS divisé par la concentration de la mauvaise odeur diluée à 0.0025 mol.L⁻¹ dans de l'éthanol.
nUDM / nMO = Concentration en moles d'undécylénate de méthyle dilué à 1% dans l'éthanol divisé par la concentration en moles de la mauvaise odeur diluée dans l'éthanol. nUDM+SSPS / nMO SSPS = Concentration en moles du mélange UDM + SSPS divisé par la concentration de la mauvaise odeur diluée dans l'éthanol.

**Tableau 1**

| MOR | Ammoniaque | nMOR/nMO | Thiogéraniol | nMOR/nMO | Diméthyl trisulfure | nMOR/nMO |
|---|---|---|---|---|---|---|
| UDM | 30% | 160 | 11% | 180 | 56% | 180 |
| SSPS | 25% | 0,8 | 33% | 0,6 | 13% | 0,6 |
| UDM+SSPS | 100% | | 100% | | 100% | |

Comme il ressort de ce Tableau, les constituants de la composition selon l'invention permettent d'éliminer les mauvaises odeurs de manière synergique.

### Exemple 2 : Produits désodorisants

Les formulations suivantes ont été préparées de manière classique pour l'homme de l'art, en mélangeant les ingrédients ci-dessous dans les proportions pondérales indiquées. Dans chacune de ces formules, le parfum renfermait 1% en poids d'un mélange de 1,8-para-menthènethiol (1,8-PMT) et d'acétate de 3-mercaptohexyle (3-HMA) dilués à 0,00001% en poids dans le dipropylène glycol, dans un rapport pondéral 1,8-PMT:3-HMA de 61:39. L'undécylénate de méthyle était utilisé pur.

**Tableau 2**

| FORMULE POUR PULVERISATEUR | | |
|---|---|---|
| **CONSTITUANT** | **INCI** | **% poids** |
| ETHANOL | *Ethanol* | 91,75% |
| UNDECYLENATE DE METHYLE | *Methyl undecylenate* | 0,25% |
| PARFUM | / | 1,00% |

### FORMULE POUR BOUGIE

| **CONSTITUANT** | **INCI** | **% poids** |
|---|---|---|
| CIRE | / | 95,75% |
| UNDECYLENATE DE METHYLE | *Methyl undecylenate* | 0,25% |
| PARFUM | / | 4,00% |

### FORMULE POUR DIFFUSEUR A BATONNETS

**Tableau 2**

| **CONSTITUANT** | **INCI** | **% poids** |
|---|---|---|
| SOLVANT | *Dipropylene Glycol Methyl Ether* | 91,75% |
| UNDECYLENATE DE METHYLE | *Methyl undecylenate* | 0,25% |
| PARFUM | / | 8,00% |

### MICROCAPSULES

On a préparé des microcapsules comprenant la composition désodorisante selon l'invention, comme décrit dans le brevet FR 3 069 252.

Pour ce faire, on a préparé une émulsion huile-dans-eau en mélangeant, dans l'ordre, 32,6g d'eau, 15,65g de tensioactif (solution à 10%) constitué d'un copolymère poly(méthylvinyléther-co-anhydride maléique) commercialisé par ASHLAND INC. sous la dénomination commerciale Gantrez^{®} AN-119 BF, 32,6 g du parfum renfermant le 1,8-PMT et le 3-HMA, 1,8g d'undécylénate de méthyle et 7,3g de prépolymère mélamine-formaldéhyde fourni par SYRIATOS SA sous la dénomination commerciale WF-70^{®}. Le mélange a été effectué sous vive agitation (7.500 tours/min) pendant 10 minutes à 70-80°C. L'agitation a été diminuée à 600 tours/min et poursuivie pendant 3h à 90°C pour former la paroi des microcapsules. Toujours sous agitation, on a alors ajouté à la suspension 0,32g de stéarate de magnésium à l'état fondu (à 95-100°C). L'agitation a été poursuivie pendant 30 min, puis la suspension a été refroidie jusqu'à température ambiante pour cristalliser le stéarate de magnésium autour des microcapsules. 0,85g d'un second tensioactif (PEG-100 stéarate) a été ajouté à la suspension, qui a été neutralisée par 2,7g d'une solution aqueuse de triéthanolamine à 30% en poids. 3,5g d'un piégeur de formaldéhyde, constitué d'éthylène urée, ont ensuite été ajoutés à la suspension.

On a ainsi obtenu une suspension aqueuse de microcapsules de parfum à structure bicouche, qui peuvent être incorporées dans une composition détergente, cosmétique ou pharmaceutique ou dans des matériaux textiles, du papier ou du carton, de préférence dans une composition détergente.

### GALET EN SILICONE

Conformément à l'enseignement de la demande WO 2019/137894, une pièce en silicone de 8,2 g a été immergée pendant 6 h dans une solution constituée de 7,5% en poids de undécylénate de méthyle, 74 % en poids du mélange parfum/SSPS (99:1) décrit à l'Exemple 1 ci-dessus et 18,5% en poids d'une solution constituée à 50% en poids d'isopropyl myristate et 50% en poids de dioctyle adipate, pour obtenir un matériau constitué à 0,5 % en poids d'undécylénate de méthyle, 5,2 % en poids de parfum, 0,65% en poids d'isopropyl myristate, 0,65 % en poids de dioctyle adipate et 93 % en poids de silicone.

La pièce en silicone imprégnée ainsi obtenue peut être utilisée comme galet à poser dans une armoire pour désodoriser le linge.

Les formulations ci-dessus, utilisées dans les conditions usuelles, permettent de limiter, voire d'empêcher, la diffusion des mauvaises odeurs présentes dans l'air ambiant.

### Exemple 3 : Mise en évidence de l'effet désodorisant en conditions réelles

On a testé plusieurs dispositifs désodorisants incorporant les formulations présentées à l'Exemple 2, par comparaison avec les mêmes formulations ne renfermant que l'undécylénate de méthyle (UDM) ou que le parfum renfermant le mélange 1,8-PMT + 3-HMA. Ces essais ont été réalisés dans trois cabines sensorielles de 3 m³ dont la qualité d'air répondait à la norme ISO-8, ayant un taux d'humidité de 45-55%, une température de 22 ± 3°C et une pression et une qualité de l'air contrôlées (filtration G4 F7). Une mauvaise odeur (MO) a été introduite dans chaque cabine. Elle était constituée d'un mélange de 5 à 100 ppm de skatole, de 5 à 100 ppm de trisulfure de diméthyle, de 5 à 100 ppm dethiogéraniol, de 80 à 4000 ppm d'ammoniac et de 30 à 600 ppm d'acide isovalérique. Le temps d'élimination de la mauvaise odeur a été évalué dans chaque cas par un panel entraîné et la moyenne des résultats a été calculée.

### Exemple 3A: Pulvérisateur d'ambiance

### Protocole

Une quantité définie (1,35 ± 0,03g) de produit (UDM ou Parfum ou UDM + Parfum), puis une quantité définie de mauvaise odeur (3 pressions soit 0.174g à 0.180g), a été vaporisée dans chacune des cabines. Chaque panéliste a senti à son tour les 3 cabines et évalué la mauvaise odeur MO (sur une échelle de 0 à 10). Cette évaluation a été réalisée toutes les 5 minutes jusqu'à disparition totale de la mauvaise odeur.

### Résultats

Les résultats obtenus sont rassemblés dans le Tableau 3 ci-dessous.

**Tableau 3**

| Cabine | UDM (% pds) | Parfum (% pds) | Temps d'élimination de la MO (min) |
|---|---|---|---|
| 1 | 0 | 1 | 15 |
| 2 | 0,5 | 0 | 35 |
| 3 | 0,5 | 1 | 7 |

Ces résultats montrent que les constituants de la composition selon l'invention permettent d'éliminer les mauvaises odeurs de manière synergique et rapide.

### Exemple 3B: Diffuseur à bâtonnets

### Protocole

3 diffuseurs à bâtonnets ont été préparés la veille du test. Pour ce faire, 4 bâtonnets de 22 cm ont été plongés chacun dans un flacon contenant 20 g d'une solution désodorisante, correspondant respectivement à la solution d'undécylénate de méthyle, à la solution de parfum ou au mélange des deux. Chacun des diffuseurs a été placé dans une cabine 20 minutes avant le test. Pour débuter le test, on a vaporisé une quantité définie de mauvaise odeur (3 pressions soit 0.174g à 0.180g) dans chaque cabine. Chaque panéliste a senti à son tour les 3 cabines et évalué la mauvaise odeur (sur une échelle de 0 à 10). Cette évaluation a été réalisée toutes les 5 minutes jusqu'à disparition totale de la mauvaise odeur.

### Résultats

Les résultats obtenus sont rassemblés dans le Tableau 4 ci-dessous.

**Tableau 4**

| Cabine | UDM (% pds) | Parfum (% pds) | Temps d'élimination de la MO (min) |
|---|---|---|---|
| 1 | 0 | 8 | 25 |
| 2 | 0,25 | 0 | 15 |
| 3 | 0,25 | 8 | 10 |

Ces résultats montrent que les constituants de la composition selon l'invention permettent d'éliminer les mauvaises odeurs de manière synergique et rapide.

### Exemple 3C: Bougie

### Protocole

3 bougies ont été préparées avant le test, qui contenaient respectivement soit de l'undécylénate de méthyle, soit le parfum, soit un mélange des deux. On a allumé et placé chaque bougie dans une cabine différente, après avoir vérifié que la mèche était longue d'environ 1,1 à 1,5 cm. On a ensuite vaporisé la mauvaise odeur (3 pressions soit 0.174g à 0.180g) dans chacune des cabines. Chaque panéliste a senti à son tour les 3 cabines et évalué la mauvaise odeur (sur une échelle de 0 à 10). Cette évaluation a été réalisée toutes les 5 minutes jusqu'à disparition totale de la mauvaise odeur.

### Résultats

Les résultats obtenus sont rassemblés dans le Tableau 5 ci-dessous.

**Tableau 5**

| Cabine | UDM (% pds) | Parfum (% pds) | Temps d'élimination de la MO (min) |
|---|---|---|---|
| 1 | 0 | 2 | 20 |
| 2 | 0,25 | 0 | 25 |
| 3 | 0,25 | 2 | 15 |

Ces résultats montrent que les constituants de la composition selon l'invention permettent d'éliminer les mauvaises odeurs de manière synergique.

### Exemple 3D : Pulvérisateur pour tissus

### Protocole

Trois serviettes en coton de 15x15 cm (provenant de Cheeky Wipes) ont été placées sur un support en verre. Sur chaque serviette, à une distance de 22cm on a vaporisé (au milieu de la serviette) la mauvaise odeur et ensuite 2 sprays de solution (0.365 à 390g) à 0.25% de capsules contenant respectivement soit de l'undécylénate de méthyle, soit le parfum, soit un mélange des deux. Les serviettes ont ensuite été placées sur une surface neutre olfactivement. L'évaluation de l'intensité de la mauvaise odeur de chaque serviette à a été réalisée au bout de 10 min. Une évaluation de l'intensité de la mauvaise odeur est également réalisée après avoir frotté 10 fois chaque serviette.

### Résultats

Les résultats obtenus sont rassemblés dans le Tableau 6 ci-dessous.

**Tableau 6**

| Serviette | UDM (% pds) | Parfum (% pds) | Intensité MO avant frottement (/10) | Intensité MO après frottement (/10) |
|---|---|---|---|---|
| 1 | 2 | 38 | 6.5 | 0 |
| 2 | 2 | 0 | 6 | 5 |
| 3 | 0 | 38 | 6 | 4 |

Ces résultats montrent que la composition selon l'invention permet d'éliminer totalement les mauvaises odeurs présentes sur des tissus.

## Revendications

1. Composition renfermant : (a) du 1,8-para-menthènethiol, et (b) de l'acétate de 3-mercaptohexyle, **caractérisée en ce qu'**elle renferme en outre (c) de l'acide undécylénique ou l'un de ses dérivés.

2. Composition selon la revendication 1, **caractérisée en ce que** le 1,8-para-menthènethiol et l'acétate de 3-mercaptohexyle sont apportés par un extrait végétal ou un mélange d'extraits végétaux.

3. Composition selon la revendication 2, **caractérisée en ce que** ledit extrait végétal est obtenu par extraction à l'aide de CO₂ supercritique ou par hydrodistillation, de préférence à l'aide de CO₂ supercritique, d'une partie de plante.

4. Composition selon la revendication 2 ou 3, **caractérisée en ce que** l'extrait végétal est un extrait de Timur (*Zanthoxylum armatum*).

5. Composition selon l'une quelconque des revendications 1 à 4, **caractérisée en ce qu'**elle renferme de de 100 à 10.000 ppm, de préférence de 600 à 6.000 ppm, de 1,8-para-menthènethiol.

6. Composition selon l'une quelconque des revendications 1 à 5, **caractérisée en ce qu'**elle renferme de 100 à 10.000 ppm, de préférence de 400 à 4.000 ppm d'acétate de 3-mercaptohexyle.

7. Composition selon l'une quelconque des revendications 1 à 6, **caractérisée en ce qu'**elle renferme de 0,001 à 10% en poids, de préférence de 0,01 à 5% en poids et plus préférentiellement de 0,1 à 1% en poids, d'acide undécylénique ou de l'un de ses dérivés.

8. Composition selon l'une quelconque des revendications 1 à 7, **caractérisée en ce que** les dérivés d'acide undécylénique sont choisis parmi : un ester alkylique d'acide undécylénique, en particulier un ester d'alkyle en C₁-C₁₂ tel qu'un ester de de méthyle, d'éthyle, de propyle, d'hexyle ou de décyle ; un ester d'acide polyoxyalkylénique, notamment de polyoxyéthylène, polyoxypropylène ou poly(oxyéthylène)(oxypropylène), d'acide undécylénique, renfermant de préférence de 2 à 20 motifs polyoxyalkylène ; un sel d'acide undécylénique, en particulier un sel métallique, tel que les sels de sodium, potassium, magnésium, calcium, zinc, cuivre ou un sel d'ammonium d'acide undécylénique ; et leurs mélanges.

9. Utilisation de la composition selon l'une quelconque des revendications 1 à 8 pour éliminer les mauvaises odeurs.

10. Utilisation selon la revendication 9, **caractérisée en ce que** ladite composition constitue un additif ajouté à un produit détergent tel qu'une lessive ou un nettoyant ménager ; à un produit d'hygiène corporelle tel qu'un déodorant, un anti-transpirant, un savon ou un shampooing ; ou à un produit de coloration capillaire.

11. Utilisation selon la revendication 9, **caractérisée en ce que** la composition est utilisée dans la fabrication d'un produit désodorisant.

12. Produit désodorisant sous forme d'aérosol, de bougie, de diffuseur de parfum à mèche, à bâtonnets ou électrique, ou d'article constitué d'un matériau polymérique solide, notamment d'un élastomère de silicone, contenant une composition telle que définie dans l'une quelconque des revendications 1 à 8.

## Patentansprüche

1. Zusammensetzung, die Folgendes umfasst: (a) 1,8-para-Menthenthiol und (b) 3-Mercaptohexylacetat, **dadurch gekennzeichnet, dass** sie außerdem (c) Undecylensäure oder eines ihrer Derivate umfasst.

2. Zusammensetzung nach Anspruch 1, **dadurch gekennzeichnet, dass** 1,8-para-Menthenthiol und 3-Mercaptohexylacetat durch einen Pflanzenextrakt oder eine Mischung von Pflanzenextrakten zugeführt werden.

3. Zusammensetzung nach Anspruch 2, **dadurch gekennzeichnet, dass** der Pflanzenextrakt durch eine Extraktion mittels überkritischem CO₂ oder durch eine Wasserdampfdestillation, vorzugsweise mittels überkritischem CO₂, eines Pflanzenteils erhalten wird.

4. Zusammensetzung nach Anspruch 2 oder 3, **dadurch gekennzeichnet, dass** es sich beim Pflanzenextrakt um einen Extrakt von Timur (*Zanthoxylum armatum*) handelt.

5. Zusammensetzung nach einem der Ansprüche 1 bis 4, **dadurch gekennzeichnet, dass** sie 100 bis 10.000 ppm, vorzugsweise 600 bis 6000 ppm, 1,8-para-Menthenthiol umfasst.

6. Zusammensetzung nach einem der Ansprüche 1 bis 5, **dadurch gekennzeichnet, dass** sie 100 bis 10.000 ppm, vorzugsweise 400 bis 4000 ppm, 3-Mercaptohexylacetat umfasst.

7. Zusammensetzung nach einem der Ansprüche 1 bis 6, **dadurch gekennzeichnet, dass** sie 0,001 bis 10 Gew.-%, vorzugsweise 0,01 bis 5 Gew.-% und stärker bevorzugt 0,1 bis 1 Gew.-% Undecylensäure oder eines ihrer Derivate umfasst.

8. Zusammensetzung nach einem der Ansprüche 1 bis 7, **dadurch gekennzeichnet, dass** die Derivate von Undecylensäure ausgewählt sind aus: einem Alkylester von Undecylensäure, insbesondere einem C₁-C₁₂-Alkylester, wie einem Methyl-, Ethyl-, Propyl-, Hexyl- oder Decylester; einem Polyoxyalkylensäureester, insbesondere einem Polyoxyethylen-, Polyoxypropylen- oder Poly(oxyethylen)(oxypropylen)säureester, von Undecylensäure, der vorzugsweise 2 bis 20 Polyoxyalkyleneinheiten umfasst; einem Salz von Undecylensäure, insbesondere einem Metallsalz, wie Natrium-, Kalium-, Magnesium-, Calcium-, Zink-, Kupfersalze, oder einem Ammoniumsalz von Undecylensäure; und Mischungen davon.

9. Verwendung der Zusammensetzung nach einem der Ansprüche 1 bis 8 zur Beseitigung von schlechten Gerüchen.

10. Verwendung nach Anspruch 9, **dadurch gekennzeichnet, dass** die Zusammensetzung ein Additiv darstellt, das zu einem Detergensprodukt, wie einem Waschmittel oder einem Haushaltsreiniger; zu einem Körperpflegeprodukt, wie einem Deodorant, einem Antitranspirant, einer Seife oder einem Shampoo; oder zu einem Haarfärbeprodukt gegeben wird.

11. Verwendung nach Anspruch 9, **dadurch gekennzeichnet, dass** die Zusammensetzung zur Herstellung eines desodorierenden Produkts verwendet wird.

12. Desodorierendes Produkt in Form eines Aerosols, einer Kerze, eines Docht-, Stäbchen- oder elektrischen Duftspenders oder eines Artikels, der aus einem festen Polymermaterial, insbesondere einem Siliconelastomer, besteht, das eine Zusammensetzung gemäß der Definition in einem der Ansprüche 1 bis 8 enthält.

## Claims

1. A composition containing: (a) 1,8-para-menthenethiol and (b) 3-mercaptohexyl acetate, **characterized in that** it also contains (c) undecylenic acid or one of its derivatives.

2. The composition as claimed in claim 1, **characterized in that** the 1,8-para-menthenethiol and 3-mercaptohexyl acetate are provided by a plant extract or a mixture of plant extracts.

3. The composition as claimed in claim 2, **characterized in that** said plant extract is obtained by extraction using supercritical CO₂ or by hydrodistillation, preferably using supercritical CO₂, of a plant part.

4. The composition as claimed in claim 2 or 3, **characterized in that** the plant extract is an extract of timur (*Zanthoxylum armatum*)*.*

5. The composition as claimed in any one of claims 1 to 4, **characterized in that** it contains from 100 to 10 000 ppm, preferably from 600 to 6000 ppm, of 1,8-para-menthenethiol.

6. The composition as claimed in any one of claims 1 to 5, **characterized in that** it contains from 100 to 10 000 ppm, preferably from 400 to 4000 ppm of 3-mercaptohexyl acetate.

7. The composition as claimed in any one of claims 1 to 6, **characterized in that** it contains from 0.001% to 10% by weight, preferably from 0.01% to 5% by weight and more preferentially from 0.1% to 1% by weight, of undecylenic acid or of a derivative thereof.

8. The composition as claimed in any one of claims 1 to 7, **characterized in that** the derivatives of undecylenic acid are chosen from: an alkyl ester of undecylenic acid, in particular a C₁-C₁₂ alkyl ester such as a methyl, ethyl, propyl, hexyl or decyl ester; an ester of polyoxyalkylenic acid, notably of polyoxyethylene, polyoxypropylene or poly(oxyethylene) (oxypropylene), of undecylenic acid, preferably containing from 2 to 20 polyoxyalkylene units; a salt of undecylenic acid, in particular a metal salt, such as the sodium, potassium, magnesium, calcium, zinc or copper salts or an ammonium salt of undecylenic acid; and mixtures thereof.

9. The use of the composition as claimed in any one of claims 1 to 8, for eliminating unpleasant odors.

10. The use as claimed in claim 9, **characterized in that** said composition constitutes an additive added to a detergent product such as a laundry detergent or a household cleaning product; to a body hygiene product, such as a deodorant, an antiperspirant, a soap or a shampoo; or to a hair dye product.

11. The use as claimed in claim 9, **characterized in that** the composition is used in the manufacture of a deodorizing product.

12. A deodorizing product in the form of an aerosol, a candle, or a wick, reed or electrical fragrance diffuser, or an article consisting of a solid polymer material, notably of a silicone elastomer, containing a composition as defined in any one of claims 1 to 8.
